(19) Europäisches Patentamt
European Patent Office
Office européen des brevets

(11) EP 1 182 262 A1

(12) EUROPEAN PATENT APPLICATION

(43) Date of publication:
27.02.2002 Bulletin 2002/09

(51) Int Cl.[7]: C12P 17/04, C12N 1/20
// (C12N1/20, C12R1:01)

(21) Application number: 01119585.6

(22) Date of filing: 16.08.2001

(84) Designated Contracting States:
AT BE CH CY DE DK ES FI FR GB GR IE IT LI LU MC NL PT SE TR
Designated Extension States:
AL LT LV MK RO SI

(30) Priority: 23.08.2000 EP 00118059

(71) Applicant: Roche Vitamins AG
4070 Basel (CH)

(72) Inventors:
- Asakura, Akira
Fujisawa-shi, Kanagawa-ken 251-0032 (JP)
- Hoshino, Tatsuo
Kamakura-shi, Kanagawa-ken 248-0027 (JP)
- Shinjoh, Masako
Kamakura-shi, Kanagawa-ken 247-0061 (JP)

(74) Representative: Müller, Ingrid, Dr. et al
Grenzacherstrasse 124
CH-4070 Basel (CH)

(54) **Microbial production of l-ascorbic acid and d-erythorbic acid**

(57) A process for producing L-ascorbic acid or D-erythorbic acid or in each case its sodium, potassium or calcium salt, from 2-keto-L-gulonic acid or 2-keto-D-gluconic acid or in each case its sodium, potassium or calcium salt, involves incubating 2-keto-L-gulonic acid or 2-keto-D-gluconic acid, each as the free acid or as its sodium, potassium or calcium salt, and cells of a thermoacidophilic microorganism at temperatures from about 30°C to about 100°C and at a pH from about 1 to about 6 in a solution to form L-ascorbic acid or D-erythorbic acid or an appropriate salt thereof, and isolating said product from the solution. L-Ascorbic acid (vitamin C) is widely used not only in health care as such but also in food, animal feed, e.g. fish feed, and cosmetics. D-erythorbic acid is mainly used as an antioxidant for food additives.

EP 1 182 262 A1

## Description

The present invention concerns a novel microbial process for producing L-ascorbic acid and D-erythorbic acid and salts thereof. More specifically, the present invention concerns a novel process for producing L-ascorbic acid or D-erythorbic acid from 2-keto-L-gulonic acid or 2-keto-D-gluconic acid, respectively, or the respective salts from appropriate salts of the starting materials, using cells of a thermoacidophilic microorganism. L-Ascorbic acid (vitamin C) is widely used not only in health care as such but also in food, animal feed, e.g. fish feed, and cosmetics. D-erythorbic acid is mainly used as an antioxidant for food additives.

L-Ascorbic acid has been produced from D-glucose by the well-known Reichstein method (Helv. Chim. Acta 17, 311-328, 1934). In this multi-step method, L-ascorbic acid is produced chemically from the intermediate 2-keto-L-gulonic acid. The method has been used commercially for more than 60 years with many chemical and technical modifications to improve the efficiency of each of the steps to the intermediates D-sorbitol, L-sorbose, diacetone-L-sorbose, diacetone-2-keto-L-gulonic acid, 2-keto-L-gulonic acid and methyl 2-keto-L-gulonate, and to the final product L-ascorbic acid. The conversion of D-sorbitol to L-sorbose is the sole microbial step, the others being chemical steps. The conversion of diacetone-2-keto-L-gulonic acid into L-ascorbic acid has been performed by two different procedures: 1) deprotection to give 2-keto-L-gulonic acid, followed by esterification with methanol and base-catalyzed cyclization; and 2) acid-catalyzed cyclization to L-ascorbic acid directly from the protected or deprotected 2-keto-L-gulonic acid. These conversion processes must be performed in non-aqueous or low-aqueous reaction media. Environmentally and economically, reaction without organic solvents is preferable.

In its turn, D-erythorbic acid has been produced from D-glucose via 2-keto-D-gluconic acid, which itself can be produced by fermentation with a strain belonging to the genus *Pseudomonas*, and via methyl 2-keto-D-gluconate.

Much time and effort has been devoted to finding other methods of producing L-ascorbic acid using microorganisms. Most studies on the microbial production of L-ascorbic acid have focused on the production of the intermediate 2-keto-L-gulonic acid, in particular from L-sorbose (G. Z. Yin et al., Sheng Wu Hsueh Pao. 20, 246-251, 1980; A. Fujiwara et al., EP 213 591; T. Hoshino et al., US Patent No. 4,960,695; and I. Nogami et al., EP 221 707), from D-sorbitol (A. Fujiwara et al., EP 213 591; T. Hoshino et al., US Patent No. 5,312,741; M. Niwa et al., WO 95/23220; and S. F. Stoddard et al., WO 98/17819), or from D-glucose via 2,5-diketogluconic acid with a single, mixed or recombinant culture (T. Sonoyama et al., Appl. Environ. Microbiol. 43, 1064-1069, 1982; and S. Anderson et al., Science 230, 144-149, 1985). The 2-keto-L-gulonic acid can then be converted into L-ascorbic acid by chemical means as described above.

The involvement of a biological process for the conversion of 2-keto-L-gulonic acid ester into L-ascorbic acid has recently been reported in US Patent No. 6,022,719 (J.C. Hubbs). In this patent, there is described a process for producing L-ascorbic acid by contacting 2-keto-L-gulonic acid or an ester thereof with a hydrolase enzyme catalyst selected from the group consisting of a protease, an esterase, a lipase and an amidase. The patent exemplifies the formation of L-ascorbic acid from an ester of 2-keto-L-gulonic acid such as butyl 2-keto-L-gulonate, but not the formation of L-ascorbic acid from 2-keto-L-gulonic acid itself. It discloses, for example, that *Candida antartica* B lipase catalyzed the reaction to form 413 to 530 mg/l of methyl 2-keto-L-gulonate, but no L-ascorbic acid, from 1% (w/v) 2-keto-L-gulonic acid in the presence of 8.6% methanol at a pH of from 3.1 to 3.2 and at 38°C. Ester synthetic activity of *Candida antartica* B lipase on 2-keto-L-gulonic acid, an $\alpha$-keto-carboxylic acid, at acidic pH was apparently positive, but intramolecular ester formation by this lipase was negligible.

In addition to the hydrolase reaction, ester bond syntheses such as those for proteins (amino-esters), for fatty acid esters (carboxyl-esters) and for nucleotide chains (phospho-esters) are highly functional in cells. Even in aqueous phase, the reactions proceed unidirectionally and are seldom inhibited by the product, compared with the reverse reaction of a hydrolase. Such reaction systems require a supply of activated esters such as activated transfer ribonucleic acid (tRNA), adenosine triphosphate (ATP), acyl coenzyme A (acyl-CoA) and the like, which are generated through energy-converting metabolism in cells. "In vitro" reconstitution of the reactions needs a stoichiometric supply or regeneration system of energy donors such as ATP, which are expensive for commercial production of vitamins and fine chemicals such as L-ascorbic acid and D-erythorbic acid. Commercially, utilization of intact cells is one of the preferred methods.

As mentioned above, the chemical conversion of 2-keto-L-gulonic acid to L-ascorbic acid via 2-keto-L-gulonic acid $\gamma$-lactone is an acid-catalyzed reaction accompanied by the elimination of a water molecule. The main principle of the reaction is a carboxyl ester bond formation to afford a $\gamma$-lactone ring in a 2-keto-L-gulonic acid molecule. Therefore, especially in the aqueous phase, the final state in the equilibrium reaction is determined by the physico-chemical conditions. Productivity of L-ascorbic acid from 2-keto-L-gulonic acid by chemical conversion is considerable even in aqueous phase, but not sufficient for commercial application. Performing the process in aqueous phase or in an aqueous phase with a low content of an organic solvent is highly desirable for cost effectiveness and for complying with environmental demands. Accordingly, some biological enhancement of the chemical conversion is desirable for the production in aqueous phase. Clearly, both high temperature and acidic (low) pH are desirable reaction parameters for improving the efficiency of the chemical reaction. But, in general, such physico-chemical conditions are known to be biologically incompatible for cell living and/or cellular activity of most of the microorganisms viable under mesophilic conditions. Utilization of thermophilic or acidophilic microorganisms is well known, but there have been few examples of the use of thermoacidophilic microorganisms which have tolerance to both heat and acid.

Surprisingly, it has now been found that the conversion of 2-keto-L-gulonic acid, as the free acid or as its sodium potassium or calcium salt, to L-ascorbic acid or the appropriate salt in aqueous phase can be directly and favorably performed by thermoacidophilic microorganisms under biologically extreme conditions, i.e at high temperature and low (acid) pH. It has further been surprisingly found that the conversion of 2-keto-D-gluconic acid, as the free acid or as its sodium, potassium or calcium salt, to D-erythorbic acid or the appropriate salt in aqueous phase can also be directly and favorably performed by thermoacidophilic microorganisms under biologically extreme conditions.

Accordingly, the present invention provides a process for producing L-ascorbic acid or its sodium, potassium or calcium salt from 2-keto-L-gulonic acid or its sodium, potassium or calcium salt, or for producing D-erythorbic acid or its sodium, potassium or calcium salt from 2-keto-D-gluconic acid or its sodium, potassium or calcium salt, using thermoacidophilic microorganisms. More particularly, the process of the present invention is a process for producing L-ascorbic acid or its sodium, potassium or calcium salt from 2-keto-L-gulonic acid or its sodium, potassium or calcuim salt, or for producing D-erythorbic acid or its sodium, potassium or calcium salt from 2-keto-D-gluconic acid or its sodium, potassium or calcium salt, comprising incubating 2-keto-L-gulonic acid or 2-keto-D-gluconic acid , each as the free acid or as its sodium, potassium or calcium salt, and cells of a thermoacidophilic microorganism which is capable of producing and/or enhancing the production of L-ascorbic acid or its sodium, potassium or calcium salt from 2-keto-L-gulonic acid or its sodium, potassium or calcium salt, or respectively D-erythorbic acid or its sodium, potassium or calcium salt from 2-keto-D-gluconic acid or its sodium, potassium or calcium salt, at a high temperature, i.e. at temperatures from about 30°C to about 100°C, and at an acidic pH, i.e. at a pH from about 1 to about 6, in a solution to form L-ascorbic acid or its sodium, potassium or calcium salt or D-erythorbic acid or its sodium, potassium or calcium salt, and isolating L-ascorbic acid or its sodium, potassium or calcium salt or D-erythorbic acid or its sodium, potassium or calcium salt from the solution.

A further embodiment of the present invention is a microorganism that produces L-ascorbic acid or a salt thereof or D-erythorbic acid or a salt thereof having the following characteristics:

(a) an rDNA sequence that is at least 98.1% identical to SEQ ID NOs: 1, 2 or 3 using the Genetyx-SV/R software program;
(b) a rod-shaped morphology;
(c) a width of about 0.8 μm;
(d) an inability to grow under anaerobic conditions;
(e) exhibiting catalase activity;
(f) $\bar{\omega}$-Cycohexylic acid as its major fatty acid;
(g) an ability to grow at a pH of 3.0 and a temperature of 60°C;
(h) an inability to grow under the following conditions:

| pH | Temperature |
|---|---|
| 3.0 | 30°C |
| 6.5 | 60°C |
| 6.5 | 30°C |

(i) an ability to produce a (1) L-ascorbic acid or a salt thereof from 2-keto-L-gulonic acid or a salt thereof, (2) D-erythorbic acid or a salt thereof from 2-keto-D-gluconic acid or a salt thereof, or (3) both L-ascorbic acid or a salt thereof and D-erythorbic acid or a salt thereof from 2-keto-L-gulonic acid or a salt thereof and 2-keto-D-gluconic acid or a salt thereof, respectively.

For the sake of brevity "or its sodium, potassium or calcium salt" or an equivalent expression as applied to "2-keto-L-gulonic acid", "2-keto-D-gluconic acid", "L-ascorbic acid" or "D-erythorbic acid" will be mainly referred to hereinafter in each case as "or its salt". Moreover, any given concentrations of these acids or their salt forms will be expressed as being based on the free acid form even through a salt form may be present, unless clearly stated for the acid or its particular salt form which is present.

In the present description the term "thermophilic microorganism" generally means a microorganism which has optimal growth at a temperature above about 55°C, and the term "acidophilic microorganism" means a microorganism which has optimal growth at a pH in the acidic range, particularly below about pH 6, and no growth in the neutral area, i.e. in the pH range from about 6 to about 8. Thus in the context of the present invention the term "thermoacidophilic microorganism" means a microorganism which has both these properties, i.e. which has optimal growth at a temperature above about 55°C, and at a pH below about 6 and no growth in the pH range from about 6 to about 8. The term "thermoacidophilic microorganism" also includes for the purposes of the present invention mutants of a thermoacidophilic microorganism.

The term "growth" as used in the present invention means that a colony formation can be observed after 20 hours of incubation. The term "no growth" as used in the present invention means that no colonies are observed after incubation for 20 hours.

Normally, thermoacidophilic microorganisms exist in and are isolable from prokaryotes and are classified under both *Archaea* and *Bacteria.* In the *Archaea* domain, the genera *Sulfolobus* (T. D. Brock et al., Arch. Mikrobiol. 84, 54-68, 1972) and *Thermoplasma* (M. DeRosa et al., Phytochemistry 170, 1416-1418, 1970) are well-known as thermoacidophilic microorganisms, and the genera *Acidanus* (A. H. Segerer et al., Int. J. Syst. Bacteriol. 36, 559-564, 1986), *Desulfurolobus* (W. Zilling et al., Syst. Appl. Microbiol. 8, 197-209, 1986), *Metallosphaera* (G. Huber et al., Syst. Appl. Microbiol. 12, 38-47, 1989), *Picrophilus* (C. Schleper et al., J. Bacteriol. 177, 7050-7059, 1995) and *Stygiolobus* (A. H. Segerer et al., Int. J. Syst. Bacteriol. 41, 495-501, 1991) have also been reported as being thermoacidophilic microorganisms of the *Archaea* domain. In the *Bacteria* domain, the genera *Acidimicrobium* (D. A. Clark et al., Microbiology 142, 785-790, 1996), *Acidothermus* (F. Rainey et al., FEMS Microbiol. Lett., 108, 27-30, 1993), *Sulfobacillus* (R. S. Golovacheva et al., Microbiology 47, 658-665, 1978) and *Alicyclobacillus* (G. Darland et al., J. Gen. Microbiol. 67, 9-15, 1971; G. Deinhard et al., Syst. Appl. Microbiol. 10, 47-53, 1987) are thermoacidophilic microorganisms.

The thermoacidophilic microorganisms useable in the present invention are any thermoacidophilic microorganisms which are capable of producing and/or enhancing the production of L-ascorbic acid or its salt from 2-keto-L-gulonic acid or its salt, or D-erythorbic acid or its salt from 2-keto-D-gluconic acid or its salt.

The thermoacidophilic microorganisms used in the present invention can be obtained from any kind of natural sources thereof, including soils and hot spring water, and from artificial sources thereof, including processed acidic foods and beverages, e.g. fruit juices and mixed fruit/vegetable juices.

The more extreme the conditions, i.e. the higher the temperature and the lower (more acid) the pH, under which any particular thermoacidophilic microorganism displays tolerance, the more preferably this microorganism is used in the process of the present invention. Besides tolerance to heat and acidity, thermoacidophilic microorganisms which are also tolerant to a high concentration, i.e. from about 5% to about 20% (w/v), of 2-keto-L-gulonic acid or its salt, or of 2-keto-D-gluconic acid or its salt, in solution when incubated at high temperature and acidic pH are also more preferably used in the process. In addition, thermoacidophilic microorganisms with the aerobic and chemoorganotrophic characteristics described in the present invention are preferable for the efficient, i.e. rapid, production of cells.

Preferred thermoacidophilic microorganisms are those derived from prokaryotes, including bacteria and archaea. More preferred microorganisms are thermoacidophilic bacteria. Especially preferred thermoacidophilic microorganisms are bacteria belonging to the genus *Alicyclobacillus.*

Among the thermoacidophilic bacteria, the genus *Alicyclobacillus* embraces most of the strictly aerobic, spore-forming, rod-shaped and chemoorganotrophic bacteria. These microorganisms were initially assigned to the genus *Bacillus.* However, phylogenetic analysis based on sequence comparisons of the 16S rRNA gene has shown that the genus *Alicyclobacillus* belongs to a distinct line of descent within the low G + C Gram-positive lineage of the *Bacillus* (J. D. Wisotzkey et al., Int. J. Syst. Microbiol. 42, 263-269, 1992). The three validly named species of the genus *Alicyclobacillus (A.)* are: *A. acidocaldarius* (DSM $446^T$, G. Darland et al., J. Gen. Microbiol. 67, 9-15, 1971), *A. acidoterrestris* (DSM $3922^T$, G. Deinhard et al., Syst. Appl. Microbiol. 10, 47-53, 1987) and *A. cycloheptanicus* (DSM $4006^T$, G. Deinhard et al., Syst. Appl. Microbiol. 10, 68-73, 1987). Besides sequence comparisons of the 16S rRNA genes, the most distinguishable characteristic of these microorganisms is the possession of structural units of ω-cyclohexyl fatty acids (ω-cyclohexylundecanoic acid, ω-cyclohexyltridecanoic acid) or of ω-cycloheptyl fatty acids (ω-cycloheptylundecanoic acid, ω-cycloheptyltridecanoic acid) (L. Albuquerque et al., Int. J. Syst. Evol. Microbiol. 50, 451-457, 2000). Several strains with the characteristics of the genus *Alicyclobacillus* have been isolated so far from acidic soils within geothermal areas and from certain non-geothermal soils. In addition to soil samples, they have also been isolated from many acidic beverages as spoilage bacteria (G. Cerny et al., Z Lebens Unters Forsch 179, 224-227, 1984; K. Yamazaki et al., Biosci. Biotech. Biochem. 60, 543-545, 1996; M. Niwa et al., Japanese Patent Publication (Kokai) No. 140696/1996). Recently, a wide diversity of genospecies among the genus *Alicyclobacillus* has been proposed in addition to the three validly named species (A. Hiraishi et al., J. Gen. Appl. Microbiol. 43, 295-304, 1997; L. Albuquerque et al., Int. J. Syst. Evol. Microbiol. 50, 451-457, 2000)

Preferred thermoacidophilic microorganisms used in the present invention have the following characteristics:

1) Thermoacidophilic growth:
   Showing growth at pH 3.0 at 60°C in 20 hours, but showing no growth at pH 3.0 at 30°C, at pH 6.5 at 30°C and at pH 6.5 at 60°C, in each case in 20 hours.
2) ω-cyclohexyl fatty acids:

Possessing ω-cyclohexyl fatty acid structural units according to gas chromatography-mass spectrometry (GC/MS) analysis.

3) 16S rRNA sequence similarity:

Phylogenic analysis of 16S genes coding for rRNA sequences confirms the allocation to the genus *Alicyclobacillus.*

The thermoacidophilic microorganisms used in the present invention can be obtained from natural and artificial sources, as indicated above, or commercially from culture depositories. For isolating the microorganisms from such natural and artificial sources the appropriate microorganism source, such as a natural source soil or hot spring water, or an artificial source processed acidic food or beverage, is conveniently cultured in an aqueous medium and/or on a solid medium supplemented with appropriate nutrients under aerobic conditions. The cultivation is conveniently conducted at temperatures above about 40°C and at pHs below about 5, preferably above about 50°C and below about pH 4, and more preferably above about 55°C and below about pH 3.5. While the cultivation period varies depending upon the pH, temperature and nutrient medium used, a period of 12 hours to several days will generally give favorable results.

Especially preferred thermoacidophilic microorganisms belonging to the genus *Alicyclobacillus* and used in the present invention are *Alicyclobacillus* sp. DSM No. 13652 and DSM No. 13653 which are obtainable from the Deutsche Sammlung von Mikroorganismen und Zellkulturen GmbH (DSMZ), Mascheroder Weg 1b, D-38124 Braunschweig, Germany, and the new strains, *Alicyclobacillus* sp. NA-20 and NA-21, which were isolated from a soil sample collected at Iwate Prefecture, Japan, and *Alicyclobacillus* sp. FJ-21, which was isolated from a commercial acidic beverage, in particular a fruit juice, purchased at Kamakura-shi, Kanagawa Prefecture, Japan.

All five especially preferred thermoacidophilic microorganisms were deposited under the Budapest Treaty on August 17, 2000 at the DSMZ and were allotted the following accession numbers:

*Alicyclobacillus* sp. : DSM No. 13652
*Alicyclobacillus* sp. : DSM No. 13653
*Alicyclobacillus* sp. NA-20 : DSM No. 13649
*Alicyclobacillus* sp. NA-21 : DSM No. 13650
*Alicyclobacillus* sp. FJ-21 : DSM No. 13651

The last three mentioned thermoacidophilic microorganisms, being new, represent a further aspect of the present invention.

In the present invention, and as indicated above, mutants of the above mentioned thermoacidophilic microorganisms can also be used. The mutant of a microorganism used in the present invention can be induced by treating a wild type strain with a mutagen such as irradiation by ultraviolet rays, X-rays, γ-rays or contact with nitrous acid or other suitable mutagens. A mutant may also be obtained by isolating a clone occurring by spontaneous mutation thereof, which may be effected by methods known by skilled artisans for such purposes. Many of these methods have been described in specialized publications, for example "Chemical Mutagens" edited by Y. Tajima, T. Yoshida and T. Kada, Kodansha Scientific Inc., Tokyo, Japan, 1973.

Moreover, biologically and taxonomically homogeneous cultures of *Alicyclobacillus* sp. DSM No. 13652, DSM No. 13653 , NA-20 (DSM No. 13649), NA-21 (DSM No. 13650) or FJ-21 (DSM No. 13651) can be used. Said term "biologically and taxonomically homogeneous cultures" in the present invention means cultures showing the following biological and taxonomical characteristics:

- growth: aerobic and thermoacidophilic
- spore: forming
- cell morphology: rod-shaped
- major fatty acids: ω-cyclohexyl fatty acids
- phylogenetical position: most close (more than 90% identity in the nucleotide sequence of 16S rRNA gene) to the strains classified in the genus *Alicyclobacillus*, such as *Alicyclobacillus* sp. DSM No. 13652, *A.* sp. UZ-1, *A.* sp. MIH-2, *A.* sp. KHA-31 and *A. acidocaldarius* DSM446$^T$, whereby the identity in the nucleotide sequence is defined using the Nucleotide Sequence Homology program (Genetyx-SV/R, version 4.0, Software Development Co., Tokyo, Japan) with default conditions (unit size to compare = 1)

The above mentioned thermoacidophilic microorganisms can be used in any form, in particular as intact cells, modified cells or immobilized cells. Methods for immobilizing cells are well known in the art (see for example W. M. Fogarty et al., Microbial Enzymes and Biotechnology, 2nd Edition, Elsevier Applied Science, pp. 373-394 (1983), and Japanese Patent Publication No. 61265/1994).

The screening of thermoacidophilic microorganisms to assess their suitability for use in the process of the present invention can be performed by the following method:

The appropriate microorganism source is cultured in an aqueous medium containing the substrate 2-keto-L-gulonic acid or its salt or 2-keto-D-gluconic acid or its salt and supplemented with appropriate nutrients under moderately aerobic conditions, i.e. under aerobic incubation without enforced aeration or vigorous agitation. A concentration of the substrate, 2-keto-L-gulonic acid or its salt, or 2-keto-D-gluconic acid or its salt, for carrying out the cultivation may be from about 3% (w/v) to about 20% (w/v), preferably from about 4% (w/v) to about 18% (w/v), and more preferably from about 5% (w/v) to about 16% (w/v). The incubation may be conducted at pHs from about 0.5 to about 4.0, preferably from about 1.0 to about 3.5, and more preferably from about 1.5 to about 3.0, and at temperatures from about 45°C to about 90°C, preferably from about 50°C to about 85°C, and more preferably from about 55°C to about 80°C. While the incubation period varies depending upon the pH, temperature and nutrient medium used, a period of about 12 hours to several days will generally give favorable results. After the incubation, the suitability of the use of the screened thermoacidophilic microorganism in the process of the present invention may be assessed by its degree of productivity (high accumulation of the product) of L-ascorbic acid or its salt, or D-erythorbic acid or its salt, compared with the productivity in a "blank" incubation, i.e. without the thermoacidophilic microorganism, whereas an over 2-fold increase of productivity over the productivity in a "blank" incubation is preferred.

In the incubation described above, the presence of a high concentration of the substrate, 2-keto-L-gulonic acid or its salt, or 2-keto-D-gluconic acid or its salt, in addition to a high temperature and an acidic pH, may represent an extreme physico-chemical condition even for a thermoacidophilic microorganism. The tolerance to 2-keto-L-gulonic acid or its salt, or 2-keto-D-gluconic acid or its salt, at high temperature and an acidic pH may be an important characteristic of the thermoacidophilic microorganisms used in the process of the present invention for retention (maintenance) of the living cell state.

The incubation for the production and/or enhancing the production of L-ascorbic acid or its salt from 2-keto-L-gulonic acid or its salt, or D-erythorbic acid or its salt from 2-keto-D-gluconic acid or its salt, with cells of a thermoacidophilic microorganism in the process of the present invention is effected in solution in an aqueous phase. The solvent for the aqueous phase is preferably water alone, i.e. without any added other solvent(s). If an additional solvent is used, however, this is preferably a lower alkanol such as methanol. The incubation for the production and/or enhancing the production of L-ascorbic acid from 2-keto-L-gulonic acid, or D-erythorbic acid from 2-keto-D-gluconic acid, each of these products or substrates being present as the free acid or the respective sodium, potassium or calcium salt, requires nutrients such as assimilable carbon sources, digestible or assimilable nitrogen sources and inorganic substances, trace elements, vitamins, L-amino acids and other growth promoting factors. As assimilable carbon sources, D-glucose, sucrose, D-glucono-δ-lactone, starch and the like can be employed. Various organic or inorganic substances may be employed as nitrogen sources, such as yeast extract, meat extract, peptone, casein, corn steep liquor, urea, amino acids, nitrates, ammonium salts such as ammonium sulfate, and the like. As inorganic substances magnesium sulfate, potassium phosphate, sodium chloride, potassium chloride, calcium chloride, and the like may be employed. Furthermore, as trace elements sulfates, hydrochlorides or phosphates of calcium, magnesium, zinc, manganese, cobalt and iron may be employed. Particularly suitable as inorganic salts are monopotassium phosphate, magnesium sulfate, ferrous sulfate and manganese sulfate. And, if necessary, conventional nutrient factors or an antifoaming agent, such as animal oil, vegetable oil or mineral oil, can be added.

The conditions of the incubation may vary depending on the species and generic character of the thermoacidophilic microorganism employed. The incubation is effected at what is considered to be a high temperature for an incubation, i.e. at temperatures from about 30°C to about 100°C, preferably from about 40°C to about 95°C, and most preferably from about 55°C to about 95°C, at an acidic pH, particularly at a pH from about 1.0 to about 6.0, preferably from about 1.0 to about 4.5, and most preferably from about 1.5 to about 3.0, under an aerobic condition. Normally, an incubation period ranging from about 1 to about 100 hours is sufficient.

The suitable initial concentration of 2-keto-L-gulonic acid or its salt or of 2-keto-D-gluconic acid or its salt, as appropriate, for the incubation depends on the particular thermoacidophilic microorganism used. However, a concentration of 2-keto-L-gulonic acid or its salt, or of 2-keto-D-gluconic acid or its salt, in each case based on the (equivalent) amount of free acid, of from about 5% (w/v) to about 20% (w/v) is generally used. This concentration is preferably from about 10% (w/v) to about 15% (w/v).

The process of the present invention shows the following characteristics:

a) Specific production rate of L-ascorbic acid or its salt:

The specific production rate for L-ascorbic acid is, for example (in the presence of 8% (w/v) 2-keto-L-gulonic acid, the substrate, and 2.5 g/L L-ascorbic acid, the product, at 59°C and pH 2.5 for 20 hours by the strain NA-21 (DSM No. 13650)) estimated to be about 2.3 mg of L-ascorbic acid / mg of crude cellular proteins / hour. This is based on the results given in Example 7, hereinafter.

b) Product inhibition:

The production in the present invention is seldom inhibited by the product, L-ascorbic acid or its salt, or D-erythorbic acid or its salt. The production in the process of the present invention may provide higher conversion yield than those obtained by reversible reactions in aqueous phase.

The L-ascorbic acid or its salt, or D-erythorbic acid or its salt, formed in solution may be separated and purified by conventional methods known in the art. If the product is the sodium, potassium or calcium salt of the respective acid, this salt may, if desired, be converted into the respective free acid by conventional methods known in the art. In each case isolation of the product may be effected by methods relying upon the differences in properties between the product and impurities (including the non-converted substrate) such as solubility, adsorbability, an electrochemical property and the distribution coefficient between two solvents. Use of an absorbent such as an ion exchange resin is an example of a convenient process for the isolation of the product. Use of an electro-dialysis system is a further example of a convenient process for isolation of the product. If the product is insufficiently pure for its subsequent use, it may be purified by conventional methods such as recrystallization and chromatography.

L-Ascorbic acid can be produced from L-sorbose or D-sorbitol by combination of the organism having the activity for converting 2-keto-L-gulonic acid to L-ascorbic acid or 2-keto-D-gluconic acid to D-erythorbic acid with an organism having L-sorbose/L-sorbosone dehydrogenase and D-sorbitol dehydrogenase and which can convert D-sorbitol and/or L-sorbose to 2-keto L-gulonic acid (see A. Fujiwara et al., EP 213 591; T. Hoshino et al., US Patent No. 4,960,695; T. Hoshino et al., US Patent No. 5,312,741); ex. *Gluconobacter oxydans* DSM 4025 in one-step conversion with one vessel or two-step conversion with two vessels.

D-Erythorbic acid can be produced from D-glucose or D-gluconic acid by combination of the organism having the activity for converting 2-keto-L-gulonic acid to L-ascorbic acid or 2-keto-D-gluconic acid to D-erythorbic acid with an organism having D-glucose dehydrogenase (Ameyama et al., Agric Biol. Chem. 45:851-861, 1981) and/or D-gluconate dehydrogenase (Shinagawa et al., Agric Biol. Chem. 48: 1517-1522, 1984) ; ex. *Gluconobacter dioxyacetonicus* IFO 3271 and which can convert D-glucose and/or D-gluconic acid to 2-keto-D-gluconic acid in one-step conversion with one vessel or two step-conversion with two vessels.

The following Examples are illustrate the process of the present invention and are not intended to limit the scope of the invention in any way.

## Example 1

Screening of thermoacidophilic microorganisms:

A) Isolation from soil samples

Soil samples collected at an acidic hot spring area at Iwate Prefecture in Japan were used for the screening. Thermoacidophilic microorganisms were recovered from the soil samples in 0.9% (w/v) NaCl solution, and isolated by spreading the solution on 573c (pH 3.5) agar plate medium containing 0.1% (w/v) of D-glucose, 0.13% (w/v) of $(NH_4)_2SO_4$, 0.1% (w/v) of yeast extract, 0.15% (w/v) of $KH_2PO_4$, 0.025% (w/v) of $MgSO_4 \cdot 7H_2O$, 0.007% (w/v) of $CaCl_2 \cdot 2H_2O$ and 2% (w/v) of agar (the pH was adjusted by 6N $H_2SO_4$; D-glucose and agar were sterilized separately). After incubation at 60°C for 20 hours, single colonies grown at pH 3.5 and 60°C were randomly collected and purified by transferring them three times onto the same agar plate medium under the same conditions. The resulting isolated microorganisms were numbered and designated as belonging to the "NA" series. For example, one such isolated microorganism was designated as NA-20, another as NA-21.

B) Isolation from acidic beverages

Various commercial acidic beverages, i.e. fruit juice products and mixed fruit/vegetable juice products, were subjected to experiments for isolating thermoacidophilic microorganisms therefrom. In each case 1 ml of the commercial product was centrifuged and the resulting pellet was washed with 1 ml of sterilized distilled water to obtain a washed pellet. The pellet was suspended in 0.1 ml of sterilized distilled water, and the suspension was spread on an agar plate of medium 573c. The plate was then incubated at 60°C for 1 to 3 days to observe single colonies, which were purified by transferring them three times onto the same agar plate medium under the same conditions. The resulting isolated microorganisms were numbered and designated as belonging to the "FJ" series. For example, one such isolated microorganism was designated as FJ-21.

Example 2

Screening of NA- and FJ-series isolated microorganisms for production of L-ascorbic acid from 2-keto-L-gulonic acid

Screening for production of L-ascorbic acid from 2-keto-L-gulonic acid was performed by the following living cell reaction system. Compositions of LM101 type media, mineral mixture [MM], vitamin mixture [VM] and amino acid mixture [AM] are listed in Tables 1 below.

Tables 1

Table 1a: Media (salt base)

| | LM101c | LM101d |
|---|---|---|
| D-glucose | variable | variable |
| $(NH_4)_2SO_4$ | 4 | 1.3 |
| $KH_2PO_4$ | 1.5 | 1.5 |
| $MgSO_4 \cdot 7H_2O$ | 0.25 | 0.25 |
| NaCl | 0.1 | 0.1 |
| KCl | 0.1 | 0.1 |
| $CaCl_2 \cdot 2H_2O$ | 0.07 | 0.07 |
| | | [g/L] |
| pH | 2.5[a] | 2.5[b] |

a: after addition of sodium 2-keto-L-gulonate monohydrate, pH is adjusted by 6N $H_2SO_4$ after addition of 2-keto-L-gulonic acid, pH is adjusted by 6N KOH

Table 1b

| Mineral Mixture [x 1000 conc.] | |
|---|---|
| $CuSO_4 \cdot 5H_2O$ | 620 |
| $FeSO_4 \cdot 5H_2O$ | 600 |
| $MnSO_4 \cdot 5H_2O$ | 600 |
| $ZnSO_4 \cdot 5H_2O$ | 600 |
| $CoCl_2 \cdot 5H_2O$ | 790 |
| $Na_2MoO_4 \cdot 5H_2O$ | 700 |
| [mg/L] | |

Table 1c

| Vitamin Mixture [x 100 conc.] | |
|---|---|
| Biotin | 100 |
| Ca(+)-Pantothenate | 100 |
| Folic acid | 100 |
| Inositol | 200 |
| Pyridoxal phosphate$\cdot H_2O$ | 100 |
| Riboflavin | 10 |

Table 1c (continued)

| Vitamin Mixture [x 100 conc.] | |
|---|---|
| Thiamine·HCl | 100 |
| Nicotinamide | 100 |
| [mg/L] | |

Table 1d

| L-Amino Acid Mixture [x 5 conc.] | |
|---|---|
| Arginine·HCl | 630 |
| Cystine | 120 |
| Glutamine | 1460 |
| Histidine·HCl·$H_2O$ | 210 |
| Isoleucine | 260 |
| Leucine | 260 |
| Lysine·HCl | 360 |
| Methionine | 76 |
| Phenylalanine | 165 |
| Threonine | 240 |
| Tryptophan | 50 |
| Tyrosine | 180 |
| Valine | 230 |
| [mg/L] | |

The thermoacidophilic microorganisms of the NA and FJ series, isolated as described in Example 1, were grown on 573c agar plate medium (pH 3.5, 60°C, 15 hours) and inoculated into medium LM101c-plus ["plus" means supplementation with MM (x 1 conc.), VM (x 0.1 conc.) and AM (x 0.01 conc.)] containing 0.25% (w/v) of D-glucose. After aerobic cultivation in test tubes (60°C, 8 hours), the resulting cells were collected by centrifugation, and were used as seeding cells after suspending in LM101c salt base (about 25 of optical density at 660 nm [OD660]). The cells were inoculated at the final OD660 of 0.2 into 0.8 ml of LM101c-plus medium (pH 2.5) containing 6% (w/v) of sodium 2-keto-L-gulonate monohydrate and 0.2% (w/v) of D-glucose. The resulting cell reaction mixture was incubated at 59°C under a moderately aerobic condition; the test tube (2.0 ml micro test tube, Eppendorf, Germany) was incubated with a pin hole (0.65 mm) at the top with rotary shaking (120 rpm with 45 mm radius). L-Ascorbic acid production was determined by HPLC analysis: YMC-Pack Polyamine II column (ID. 4.6 x 150 mm; YMC Co., Japan) at 264 nm with the mobile-phase solvent containing 70% (v/v) of acetonitrile and 15 mM of ammonium dihydrogen phosphate at a flow rate of 1.5 ml/min. The amount of water lost by evaporation was estimated as the weight decrease during the incubation, and a compensing amount of water was added to retain the original volume. After 23 hours cultivation, the L-ascorbic acid production of each strain was compared with two control values: "aerobic blank" being the L-ascorbic acid production obtained with the same medium and conditions but without cell inoculation, and "anaerobic control" being the L-ascorbic acid production with the same medium but without cell inoculation in a closed micro test tube with argon gas. As a result of the screening, strains NA-20, NA-21 and FJ-21 were selected as potent L-ascorbic acid producers; they produced 1.07, 1.19 and 1.23 g/L of L-ascorbic acid, respectively. The amounts of L-ascorbic acid in the aerobic blank and anaerobic control were about 0.10 and 0.30 g/L, respectively.

The living-cell reaction with 2-keto-D-gluconic acid (hemicalcium salt containing 1.5 mol/mol $H_2O$, SIGMA Chemical Co., St. Louis, MO, USA) was performed under the same conditions as described above except using 1.2% (w/v) of 2-keto-D-gluconic acid instead of 6% (w/v) of sodium 2-keto-L-gulonate monohydrate as the substrate. The production of D-erythorbic acid was determined by the same HPLC analysis method. The strain NA-21 produced 0.051 g/L of D-erythorbic acid after 23 hours incubation. The amounts of D-erythorbic acid in the aerobic blank and anaerobic control were not detectable (less than 0.001 g/L).

Example 3

Taxonomy of the isolated microorganisms

The three isolated microorganisms, NA-20, NA-21 and FJ-21, were aerobic, spore-forming and rod-shaped bacteria. Phenotypic characters of the isolates are summarized in the following Table 2.

Table 2

| | NA-20 | NA-21 | FJ-21 |
|---|---|---|---|
| Shape | rod | rod | rod |
| Size | | | |
| (width, μm) | 0.8 | 0.8 | 0.8 |
| (length, μm) | 2-3 | 3 - 5 | 2 - 3 |
| Gram Stain | variable | variable | negative |
| Motility | + | + | - |
| Anaerobic Growth | - | - | - |
| Oxidase Test | - | - | - |
| Catalase Test | + | + | + |
| Major Fatty Acid | ω-Cyclohexylic acids (C17 and 19) | ω-Cyclohexylic acids (C17 and 19) | ω-Cyclohexylic acids (C17 and 19) |
| Growth at[a] | | | |
| pH Temp. (°C) | | | |
| 3.0 60 | + | + | + |
| 3.0 30 | - | - | - |
| 6.5 60 | - | - | - |
| 6.5 30 | - | - | - |

a: growth on 573c agar plate for 20 hours

The microorganisms showed growth at pH 3.0/60°C in 20 hours, but not at pH 3.0/30°C, pH 6.5/30°C and pH 6.5/60°C in 20 hours. Therefore, they were characterized as thermoacidophilic *Bacillus* group *bacteria.* GC/MS analysis on fatty acids indicated that the major components of the three isolated microorganisms were identical with those of *Alicyclobacillus acidocaldarius* DSM $466^T$ investigated as the control, suggesting that ω-cyclohexyl fatty acids were the major components of the isolated microorganisms as well as of *Alicyclobacillus acidocaldarius* DSM $466^T$. 16S rRNA gene sequences of the isolated microorganisms were determined with 16S rRNA gene kit (PE Applied Biosystems, USA; SEQ ID NOs: 1, 2 and 3 for the strain NA-20, NA-21 and FJ-21, respectively) and were subjected to the BLAST search program (J. Mol. Biol. 215 403-410, 1990; Nucleic Acids Res. 25 3389-3402, 1997). The sequence similarity analysis using the Nucleotide Sequence Homology Program (Genetyx-SV/R, version 4.0, Software Development Co., Tokyo, Japan) with default conditions (unit size to compare = 1) indicated that the isolated microorganisms could belong phylogenetically to the genus *Alicyclobacillus*. The following Table 3 shows the levels of binary sequence identity (%) in 16SrRNA gene sequences between the isolated microorganisms and reference strains including three type strains.

Table 3

| Identity (%) in 16SrRNA gene | | | | | |
|---|---|---|---|---|---|
| | | Accession No. | NA-20 | NA-21 | FJ-21 |
| *A. acidocaldarius* | DSM $446^T$ | X60742 | 96.9 | 97.0 | 98.3 |
| *A. acidoterrestris* | DSM $3922^T$ | AJ133631 | 94.0 | 94.1 | 94.5 |
| *A. cycloheptanicus* | DSM $4006^T$ | X51928 | 92.6 | 92.6 | 92.9 |
| *A.* sp. | UZ-1 | AB004579 | 99.5 | 99.6 | 97.8 |
| *A.* sp. | MIH-2 | AB004580 | 99.5 | 99.6 | 97.8 |
| *A.* sp. | KHA-31 | AB004581 | 99.2 | 99.3 | 97.6 |

Table 3 (continued)

| Identity (%) in 16SrRNA gene | | | | | |
|---|---|---|---|---|---|
| | | Accession No. | NA-20 | NA-21 | FJ-21 |
| *A.* sp. | DSM 13652 | AJ133634 | 99.0 | 99.1 | 97.6 |
| *A.* sp. | NA-20 | | 100.0 | 99.7 | 98.1 |
| *A.* sp. | NA-21 | | 99.7 | 100.0 | 98.1 |
| *A.* sp. | FJ-21 | | 98.1 | 98.1 | 100.0 |

The 16S rDNA sequences of NA-20 and NA-21 were most similar to each other (99.7%) and to the sequences of *Alicyclobacillus* sp. UZ-1, MIH-2, KHA-31 (J. Gen. Appl. Microbiol., 43, 295- 304, 1997) and *A. sp.* DSM 13652 ( 99.0 to 99.6%). The sequence of FJ-21 was most similar to those of *A. acidocaldarius* DSM $446^T$, NA-20 and NA-21 (98.1 to 98.3%). From these results, the three isolated microorganisms were classified into the genus *Alicyclobacillus* and named as *Alicyclobacillus* sp. NA-20, NA-21 and FJ-21, respectively.

Under the same screening conditions as described in Example 2, *Alicyclobacillus* sp. DSM 13652 and 13653 produced 0.91 and 0.95 g/L of L-ascorbic acid from sodium 2-keto-L-gulonate monohydrate, respectively.

Example 4

Effects of carbon/energy source and aeration

In the L-ascorbic acid production process described in Example 2, the medium containing 0.2% (w/v) of D-glucose in addition to 6% (w/v) of sodium 2-keto-L-gulonate monohydrate was used to maintain cell living. For the production using *Alicyclobacillus* sp. NA-21, addition of D-glucono-δ-lactone [0.1% (w/v)], sucrose [0.1% (w/v)] or soluble starch [1% (w/v) instead of D-glucose resulted in almost the same production of L-ascorbic acid, but no addition of carbon/energy source resulted in no additional production over the aerobic blank (see Table 4).

Table 4

| Carbon | [% (w/v)] | Cell | L-Ascorbic Acid [g/L][a] |
|---|---|---|---|
| Sucrose | 0.1 | + | 0.69 |
| Soluble Starch | 1.0 | + | 0.65 |
| D-Glucono-δ-lactone | 0.1 | + | 0.76 |
| D-Glucose | 0.1 | + | 0.80 |
| D-Glucose | 0.025 | + | 0.66 |
| No Carbon | - | + | 0.10 |
| Aerobic Blank | - | - | 0.09 |

a: 13 hour production from 6% (w/v) of sodium 2-keto-L-gulonate·monohydrate

In the L-ascorbic acid production process described in Example 2, the production was carried out under moderately aerobic condition for cell living. Using *Alicyclobacillus* sp. NA-21, the results of carrying out the process under aerobic and anaerobic conditions were compared. The same reaction mixture in a completely closed micro test tube was used for the system under anaerobic conditions after gasification with argon gas. The aerobic conditions contributed to nearly linear production until 38 hours, but anaerobic conditions did not allow production even after 15 hours (see Tables 5).

Tables 5

| L-Ascorbic acid production (g/L) under aerobic condition | | | | |
|---|---|---|---|---|
| | Reaction Time [h] | | | |
| | 0 | 15 | 23 | 38 |
| + Cell | 0.00 | 0.80 | 1.29 | 2.10 |
| - Cell | 0.00 | 0.12 | 0.13 | 0.17 |

| L-Ascorbic acid production (g/L) under anaerobic condition | | | | |
|---|---|---|---|---|
| | Reaction Time [h] | | | |
| | 0 | 15 | 23 | 38 |
| + Cell | 0.00 | 0.79 | 0.87 | 1.05 |
| - Cell | 0.00 | 0.25 | 0.36 | 0.59 |

Example 5

L-Ascorbic acid production by using the strain NA-21 or its derivatives

L-Ascorbic acid production from 6% (w/v) of sodium 2-keto-L-gulonate monohydrate was examined by using *Alicyclobacillus* sp. NA-21. Preparation of seeding cell was carried out by the same method as described in Example 2. The cells were inoculated at the final OD660 of 0.25 into 0.8 ml of LM101c-plus medium (pH 2.5) containing 6% (w/v) of sodium 2-keto-L-gulonate monohydrate and 0.1% (w/v) of D-glucose. The resulting cell reaction mixture was incubated at 59°C under a moderately aerobic condition; the test tube was incubated with a pin hole (0.65 mm) at the top with rotary shaking (120 rpm with 45 mm radius). L-Ascorbic acid production by *Alicyclobacillus* sp. NA-21 continued linearly until 38 hours to reach to 2.23 g/L of L-ascorbic acid. Productivity was obviously more effective than that of the aerobic blank and of the anaerobic control (see Tables 6).

Tables 6

| OD 660 | | | | |
|---|---|---|---|---|
| | Reaction Time [h] | | | |
| | 0 | 15 | 23 | 38 |
| + Cell | 0.25 | 0.22 | 0.20 | 0.15 |
| - Cell Aerobic Blank | 0.00 | 0.00 | 0.00 | 0.00 |
| Anaerobic Control | 0.00 | 0.00 | 0.00 | 0.00 |

| pH | | | | |
|---|---|---|---|---|
| | Reaction Time [h] | | | |
| | 0 | 15 | 23 | 38 |
| + Cell | 2.46 | 2.46 | 2.45 | n.d. |
| - Cell Aerobic Blank | 2.46 | 2.44 | 2.43 | n.d. |
| Anaerobic Control | 2.46 | 2.44 | 2.44 | n.d. |
| n.d.: not determined | | | | |

| L-Ascorbic Acid Production [g/L] | | | | |
|---|---|---|---|---|
| | Reaction Time [h] | | | |
| | 0 | 15 | 23 | 38 |
| + Cell | 0.00 | 0.76 | 1.25 | 2.23 |
| - Cell Aerobic Blank | 0.00 | 0.12 | 0.13 | 0.17 |
| Anaerobic Control | 0.00 | 0.25 | 0.36 | 0.59 |

Two derivatives from the original strain, *Alicyclobacillus* sp. NA-21, MA-10 and MB-6 were sequentially obtained by a conventional mutation followed by the selection steps described below. From the original strain, MA-10 was selected as a strain having tolerance against higher 2-keto-L-gulonic acid concentration (apparently about 1% (w/v) improvement from 10% (w/v) at 60°C and pH 2.5) after ultraviolet irradiation. Subsequently, from MA-10, MB-6 was selected as a strain having tolerance against higher temperature (apparently about 2°C improvement from 60°C at 11% (w/v) of 2-keto-L-gulonic acid and pH 2.5) after treatment with N-methyl-N'-nitro-N-nitrosoguanidine. L-Ascorbic acid productivities were compared among the original strain, MA-10 and MB-6 by the same living-cell reaction described above at 62°C and pH 2.5, using 11% (w/v) of 2-keto-L-gulonic acid and medium containing x 0.5 conc. of LM101d salt base, 0.25%(w/v) of D-glucose, x 0.1 conc. of MM, x 0.05 conc. of VM and x 0.005 conc. of AM. The original strain, MA-10 and MB-6 produced 2.02, 2.22 and 2.80 g/L of L-ascorbic acid at 23 hours, respectively. The aerobic blank and the anaerobic control produced 1.63 and 1.94 g/L of L-ascorbic acid, respectively.

Example 6

L-Ascorbic acid production from 8% (w/v) 2-keto-L-gulonic acid with feeding of cells and D-glucose

L-Ascorbic acid production from 8% (w/v) 2-keto-L-gulonic acid with feeding of cells and D-glucose was examined by using *Alicyclobacillus* sp. NA-21. Preparation of seeding cell was carried out by the same method as described in Example 2. The cells were inoculated at the final OD660 of 0.25 into 0.8 ml of LM101d-plus medium (pH 2.5) containing 8% (w/v) of 2-keto-gulonic acid and 0.15% (w/v) of D-glucose. The resulting cell reaction mixture was incubated at 59°C under a moderately aerobic condition; the test tube was incubated with a pin hole (0.65 mm) at the top with rotary shaking (120 rpm with 45 mm radius). Seeding cells and D-glucose were supplied at 24 hours (0.25 of OD660 and 0.15% (w/v) at final concentration, respectively) as a spot feeding. L-Ascorbic acid production by *Alicyclobacillus* sp. NA-21 continued linearly until 47 hours to reach 3.70 g/L of L-ascorbic acid (see Table 7).

Table 7

| L-Ascorbic Acid Production [g/L] | | | | | | | | |
|---|---|---|---|---|---|---|---|---|
| Reaction Time (h) | 0 | 15 | 23 | 39 | 47 | 71 | 87 | 95 |
| + Cell | 0.00 | 1.13 | 1.97 | 2.99 | 3.70 | n.d. | n.d. | n.d. |
| - Cell Aerobic Blank | 0.00 | 0.23 | 0.32 | 0.35 | 0.36 | 0.39 | 0.32 | 0.28 |
| Anaerobic Control | 0.00 | 0.49 | 0.74 | 1.20 | 1.52 | 2.26 | 2.57 | 2.44 |
| n.d.: not determined | | | | | | | | |

On the other hand, productivity of the anaerobic control reached a maximum at about 2.5 g/L of L-ascorbic acid after about 80 hours.

Example 7

L-Ascorbic acid production from 8% (w/v) 2-keto-L-gulonic acid in the presence of L-ascorbic acid.

L-Ascorbic acid production from 8% (w/v) 2-keto-L-gulonic acid in the presence of 0 - 4.5 g/L of L-ascorbic acid was examined by using *Alicyclobacillus* sp. NA-21. Preparation of seeding cell was carried out by the same method as described in Example 2. The cells were inoculated at the final OD660 of 0.25 into 0.8 ml of LM101d-plus medium (pH 2.5) containing x 0.1 conc. of MM instead of x 1 conc. of MM and 8% (w/v) of 2-keto-gulonic acid and 0.20% (w/v) of D-glucose. The resulting cell reaction mixture was incubated at 59°C under moderately aerobic conditions; the test tube was incubated with a pin hole (0.65 mm) at the top with rotary shaking (120 rpm with 45 mm radius) for 20 hours. To the above medium, 0.0, 1.1, 2.3 or 4.5 g/L of L-ascorbic acid (product) was added at 0 hour (before the production). Productivity of *Alicyclobacillus* sp. NA-21 was nearly the same in the absence and presence of the product. On the other hand, productivity of the anaerobic control was gradually repressed by the presence of a higher concentration of the product (see Tables 8).

Tables 8

| From 0.0 g/L of Initial L-Ascorbic Acid | | | |
|---|---|---|---|
| | L-Ascorbic Acid [g/L] Reaction Time [h] | | Net Production * 20 h minus 0 h |
| | 0 | 20 | [g/L] |
| + Cell | 0 | 1.81 | 1.81 |
| - Cell Aerobic Blank | 0 | 0.44 | 0.44 |

Tables 8 (continued)

| From 0.0 g/L of Initial L-Ascorbic Acid | | | |
|---|---|---|---|
| | L-Ascorbic Acid [g/L] Reaction Time [h] | | Net Production * 20 h minus 0 h |
| | 0 | 20 | [g/L] |
| Anaerobic Control | 0 | 0.83 | 0.83 |

| From 1.3 g/L of Initial L-Ascorbic Acid | | | |
|---|---|---|---|
| | L-Ascorbic Acid [g/L] Reaction Time [h] | | Net Production 20 h minus 0 h |
| | 0 | 20 | [g/L] |
| + Cell | 1.32 | 3.16 | 1.84 |
| - Cell Aerobic Blank | 1.32 | 0.80 | -0.52 |
| Anaerobic Control | 1.32 | 1.75 | 0.43 |

| From 2.5 g/L of Initial L-Ascorbic Acid | | | |
|---|---|---|---|
| | L-Ascorbic Acid [g/L] Reaction Time [h] | | Net Production 20 h minus 0 h |
| | 0 | 20 | [g/L] |
| + Cell | 2.48 | 4.33 | 1.85 |
| - Cell Aerobic Blank | 2.48 | 1.55 | -0.93 |
| Anaerobic Control | 2.48 | 2.7 | 0.22 |

| From 4.6 g/L of Initial L-Ascorbic Acid | | | |
|---|---|---|---|
| | L-Ascorbic Acid [g/L] Reaction Time [h] | | Net Production 20 h minus 0 h |
| | 0 | 20 | [g/L] |
| + Cell | 4.55 | 6.64 | 2.09 |
| - Cell Aerobic Blank | 4.55 | 3.02 | -1.53 |
| Anaerobic Control | 4.55 | 4.21 | -0.34 |

*: Net Production means amount of L-ascorbic acid accumulated at 20 h minus that added at 0 h.

## Claims

1. A process for producing L-ascorbic acid or its sodium, potassium or calcium salt from 2-keto-L-gulonic acid or its sodium, potassium or calcium salt, or D-erythorbic acid or its sodium, potassium or calcium salt from 2-keto-D-gluconic acid or its sodium, potassium or calcium salt, comprising incubating 2-keto-L-gulonic acid or 2-keto-D-gluconic acid, each as the free acid or as its sodium, potassium or calcium salt, and cells of a thermoacidophilic microorganism at temperatures from about 30°C to about 100°C and at a pH from about 1 to about 6 in a solution to form L-ascorbic acid or D-erythorbic acid or an appropriate salt thereof, and isolating said L-ascorbic acid, D-erythorbic acid or appropriate salt thereof from the solution.

2. The process according to claim 1 wherein the thermoacidophilic microorganism is isolated from prokaryotes.

3. The process according to claim 2 wherein the prokaryotes from which the thermoacidophilic microorganism is isolated are classified under bacteria.

4. The process according to claim 3 wherein the thermoacidophilic microorganism belongs to the bacteria genus *Alicyclobacillus*.

5. The process according to claim 4 wherein the thermoacidophilic microorganism of the genus *Alicyclobacillus* is *Alicyclobacillus* sp. DSM No. 13652, DSM No. 13653, NA-20 (DSM No. 13649), NA-21 (DSM No. 13650) or FJ-21 (DSM No. 13651) or in each case a mutant thereof.

6. The process according to claim 4 wherein the thermoacidophilic microorganism of the genus *Alicyclobacillus* is a biologically and taxonomically homogeneous culture having the identifying characteristics of *Alicyclobacillus* sp. DSM No. 13652, DSM No. 13653, NA-20 (DSM No. 13649), NA-21 (DSM No. 13650) or FJ-21 (DSM No. 13651).

7. The process according to any one of claims 1 to 6 wherein the solution contains water as the solvent.

8. The process according to any one of claims 1 to 7 wherein the incubation is effected under an aerobic condition.

9. The process according to any one of claims 1 to 8 wherein the incubation is effected under an aerobic condition and in the presence of nutrients.

10. A process according to any one of claims 1 to 9 wherein the concentration of the substrate, 2-keto-L-gulonic acid, 2-keto-D-gluconic acid or in each case the sodium, potassium or calcium salt thereof in the solution is from about 5% (w/v) to about 20% (w/v), preferably from about 10% (w/v) to about 15% (w/v), based on the amount of free acid.

11. A process according to any one of claims 1 to 10 wherein the incubation is effected at a temperature from about 40 to about 95°C, preferably from about 55 to about 95°C.

12. A process according to any one of claims 1 to 11 wherein the incubation is effected at a pH from about 1.0 to about 4.5, preferably from about 1.5 to about 3.0.

13. *Alicyclobacillus* sp. NA-20 (DSM No. 13649), *Alicyclobacillus* sp. NA-21 (DSM No. 13650) and *Alicyclobacillus* sp. FJ-21 (DSM No. 13651).

European Patent Office

# EUROPEAN SEARCH REPORT

Application Number
EP 01 11 9585

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | EP 1 026 257 A (HOFFMANN LA ROCHE)<br>9 August 2000 (2000-08-09)<br>* the whole document * | 1-3,7-12 | C12P17/04<br>C12N1/20<br>//(C12N1/20, C12R1:01) |
| D,A | WO 97 43433 A (EASTMAN CHEM CO)<br>20 November 1997 (1997-11-20)<br>* examples 14,15 * | 1 | |
| D,X | WISOTZKEY J D ET AL: "COMPARATIVE SEQUENCE ANALYSES ON THE 16S RRNA RDNA OF BACILLUS-ACIDOCALDARIUS BACILLUS-ACIDOTERRESTRIS AND BACILLUS-CYCLOHEPTANICUS AND PROPOSAL FOR CREATION OF A NEW GENUS ALICYCLOBACILLUS NEW-GENUS"<br>INTERNATIONAL JOURNAL OF SYSTEMATIC BACTERIOLOGY,<br>vol. 42, no. 2, 1992, pages 263-269, XP001041904<br>ISSN: 0020-7713<br>* the whole document * | 13 | |
| | | | TECHNICAL FIELDS SEARCHED (Int.Cl.7) |
| D,X | HIRAISHI AKIRA ET AL: "Phylogenetic characterization of a new thermoacidophilic bacterium isolated from hot springs in Japan."<br>JOURNAL OF GENERAL AND APPLIED MICROBIOLOGY,<br>vol. 43, no. 5, October 1997 (1997-10), pages 295-304, XP001041901<br>ISSN: 0022-1260<br>* the whole document * | 13 | C12N<br>C12P |

The present search report has been drawn up for all claims

| Place of search | Date of completion of the search | Examiner |
|---|---|---|
| THE HAGUE | 12 December 2001 | Lejeune, R |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 01 11 9585

This annex lists the patent family members relating to the patent documents cited in the above-mentioned European search report.
The members are as contained in the European Patent Office EDP file on
The European Patent Office is in no way liable for these particulars which are merely given for the purpose of information.

12-12-2001

| Patent document cited in search report | Publication date | Patent family member(s) | Publication date |
|---|---|---|---|
| EP 1026257 A | 09-08-2000 | BR 0000073 A | 26-09-2000 |
| | | CN 1263950 A | 23-08-2000 |
| | | EP 1026257 A1 | 09-08-2000 |
| | | JP 2000210094 A | 02-08-2000 |
| | | US 6146860 A | 14-11-2000 |
| WO 9743433 A | 20-11-1997 | US 5817490 A | 06-10-1998 |
| | | AU 3075697 A | 05-12-1997 |
| | | BR 9709099 A | 03-08-1999 |
| | | CN 1225686 A | 11-08-1999 |
| | | EP 0938582 A1 | 01-09-1999 |
| | | JP 2001505042 T | 17-04-2001 |
| | | WO 9743433 A1 | 20-11-1997 |
| | | US 6022719 A | 08-02-2000 |
| | | US 6136575 A | 24-10-2000 |
| | | US 6271006 B1 | 07-08-2001 |

EPO FORM P0459

For more details about this annex : see Official Journal of the European Patent Office, No. 12/82

## Sequence Listing

<110> Roche Vitamins AG

<120> Microbial process for producing L-ascorbic acid and
D-erythorbic acid

<130> Alicyclobacillus NA20, 21, FJ21 16S nuc

<140>
<141>

<150> EP Application No. 00118059.5
<151> 2000-08-23

<160> 3

<170> PatentIn Ver. 2.1

<210> 1
<211> 1529
<212> DNA
<213> Alicyclobacillus sp.

<220>
<221> rRNA
<222> (1)..(1529)
<223> NA-20 partial 16SrRNA gene sequence

<400> 1
```
agagtttgat cctggctcag gacgaacgct ggcggcgtgc ctaatacatg caagtcgagc 60
gggtctcttc ggaggccagc ggcggacggg tgaggaacac gtgggtaatc tgcctttcag 120
gccggaataa cgcccggaaa cgggcgctaa tgccggatac gcccgcgagg aggcatcttc 180
ttgcgggga aggcccaatt gggccgctga gagaggagcc cgcggcgcat tagctngttg 240
gcggggtaac ggcccaccaa ggcgacgatg cgtagccgac ctgagagggt gaccggccac 300
actgggactg agacacggcc cagactccta cgggaggcag cagtagggaa tcttccgcaa 360
tgggcgcaag cctgacggag caacgccgcg tgagcgaaga aggccttcgg gttgtaaagc 420
tctgttgctc ggggagagcg gcatggggga tggaaagccc catgcgagac ggtaccgagt 480
gaggaagccc cggctaacta cgtgccagca gccgcggtaa aacgtagggg gcgagcgttg 540
tccggaatca ctgggcgtaa agggtgcgta ggcggtcgag caagtctgga gtgaaagtcc 600
atggctcaac catgggatgg ctttggaaac tgcttgactt gagtgctgga gaggcaaggg 660
gaattccacg tgtagcggtg aaatgcgtag agatgtggag gaataccagt ggcgaaggcg 720
ccttgctgga cagtgactga cgctgaggca cgaaagcgtg gggagcaaac aggattagat 780
accctggtag tccacgccgt aaacgatgag tgctaggtgt tgggggggaca caccccagtg 840
ccgaaggaaa cccaataagc actccgcctg gggagtacgg tcgcaagact gaaactcaaa 900
ggaattgacg gggcccgca caagcagtgg agcatgtggt ttaattcgaa gcaacgcgaa 960
gaaccttacc agggcttgac atccctctga cacnctcaga gatgaggggt cccttcgggg 1020
cagaggagac aggtggtgca tggttgtcgt cagctcgtgt cgtgagatgt tgggttcagt 1080
cccgcaacga gcgcaaccct tgacctgtgt taccagcgcg ttgaggcggg gactcacagg 1140
tgactgccgg cgtaagtcgg aggaaggcgg ggatgacgtc aaatcatcat gcccctgatg 1200
tcctgggcta cacacgtgct acaatgggcg gaacaaaggg aggcgaagcc gcgaggcgga 1260
gcgaaaccca aaaagccgct cgtagttcgg attgcaggct gcaactcgcc tgcatgaagc 1320
cggaattgct agtaatcgcg gatcagcatg ccgcggtgaa tacgttcccg ggccttgtac 1380
acaccgcccg tcacaccacg agagtcggca acacccgaag tcggtgaggt aacccctnng 1440
gggagccagc cgccgaaggt ggggtcgatg attgggtgaa agtcgtaaca aggtagccgt 1500
accggaaggt gcggctggat cacctcctt                                  1529
```

<210> 2
<211> 1529
<212> DNA
<213> Alicyclobacillus sp.

<220>
<221> rRNA

<222> (1)..(1529)
<223> NA-21 Partial 16SrRNA gene

<400> 2
```
agagtttgat cctggctcag gacgaacgct ggcggcgtgc ctaatacatg caagtcgagc 60
gggtctcttc ggaggccagc ggcggacggg tgaggaacac gtgggtaatc tgcctttcag 120
gccggaataa cgcccggaaa cgggcgctaa tgccggatac gcccgcgagg aggcatcttc 180
ttgcggggga aggcccaatt gggccactga gagaggagcc cgcggcgcat tagctngttg 240
gcggggtaac ggcccaccaa ggcgacgatg cgtagccgac ctgagagggt gaccggccac 300
actgggactg agacacggcc cagactccta cgggaggcag cagtagggaa tcttccgcaa 360
tgggcgcaag cctgacggag caacgccgcg tgagcgaaga aggccttcgg gttgtaaagc 420
tctgttgctc ggggagagcg gcatggggga tggaaagccc catgcgagac ggtaccgagt 480
gaggaagccc cggctaacta cgtgccagca gccgcggtaa aacgtagggg gcgagcgttg 540
tccggaatca ctgggcgtaa agggtgcgta ggcggtcgag caagtctgga gtgaaagtcc 600
atggctcaac catgggatgg ctttggaaac tgcttgactt gagtgctgga gaggcaaggg 660
gaattccacg tgtagcggtg aaatgcgtag agatgtggag gaataccagt ggcgaaggcg 720
ccttgctgga cagtgactga cgctgaggca cgaaagcgtg gggagcaaac aggattagat 780
accctggtag tccacgccgt aaacgatgag tgctaggtgt tgggggggaca caccccagtg 840
ccgaaggaaa cccaataagc actccgcctg gggagtacgg tcgcaagact gaaactcaaa 900
ggaattgacg gggggcccgca caagcagtgg agcatgtggt ttaattcgaa gcaacgcgaa 960
gaaccttacc agggcttgac atccctctga caccctcaga gatgaggggt cccttcgggg 1020
cagaggagac aggtggtgca tggttgtcgt cagctcgtgt cgtgagatgt tgggttcagt 1080
cccgcaacga gcgcaaccct tgacctgtgt taccagcgcg ttgaggcggg gactcacagg 1140
tgactgccgg cgtaagtcgg aggaaggcgg ggatgacgtc aaatcatcat gcccctgatg 1200
tcctgggcta cacacgtgct acaatgggcg gaacaaaggg aggcgaagcc gcgaggcgga 1260
gcgaaaccca aaaagccgct cgtagttcgg attgcaggct gcaactcgcc tgcatgaagc 1320
cggaattgct agtaatcgcg gatcagcatg ccgcggtgaa tacgttcccg ggccttgtac 1380
acaccgcccg tcacaccacg agagtcggca acacccgaag tcggtgaggt aacccttag 1440
gggagccagc cgccgaaggt ggggtcgatg attggggtga agtcgtaaca aggtagccgt 1500
accggaaggt gcggctggat cacctcctt 1529
```

<210> 3
<211> 1495
<212> DNA
<213> Alicyclobacillus sp.

<220>
<221> rRNA
<222> (1)..(1495)
<223> FJ-21 Partial 16SrRNA gene sequence

<400> 3
```
aggacgaacg ctggcggcgt gcctaataca tgcaagtcga gcggacctct tctgaggtca 60
gcggcggacg ggtgaggaac acgtgggtaa tctgcctttc agaccggaat aacgcccgga 120
aacgggcgct aatgccggat acgcccgcga ggaggcatct tcttgcgggg aaaggcccga 180
ttgggccgct gagagaggag cccgcggcgc attagctngt tggcggggta acggcccacc 240
aaggcgacga tgcgtagccg acctgagagg gtgaccggcc acactgggac tgagacacgg 300
cccagactcc tacgggaggc agcagtaggg aatcttccgc aatgggcgca agcctgacgg 360
agcaacgccg cgtgagcgaa gaaggccttc gggttgtaaa gctctgttgc tcggggagag 420
cggcatgggg agtggaaagc cccatgcgag acggtaccga gtgaggaagc cccggctaac 480
tacgtgccag cagccgcggt aaaacgtagg gggcgagcgt tgtccggaat cactgggcgt 540
aaagggtgcg taggcggtcg agcaagtctg gagtgaaagt ccatggctca accatgggat 600
ggctctggaa actgcttgac ttgagtgctg gagaggcaag gggaattcca cgtgtagcgg 660
tgaaatgcgt agagatgtgg aggaatacca gtggcgaagg cgccttgctg gacagtgact 720
gacgctgagg cacgaaagcg tggggagcaa acaggattag ataccctggt agtccacgcc 780
gtaaacgatg agtgctaggt gttgggggga cacaccccag tgccgaagga aacccaataa 840
gcactccgcc tggggagtac ggtcgcaaga ctgaaactca aaggaattga cgggggcccg 900
cacaagcagt ggagcatgtg gtttaattcg aagcaacgcg aagaacctta ccagggcttg 960
acatccctct gacgggtgca gagatgcacc ttcccttcgg ggcagaggag acaggtggtg 1020
catggttgtc gtcagctcgt gtcgtgagat gttgggttca gtcccgcaac gagcgcaacc 1080
cttgacctgt gttaccagcg cgntanggcg gggactcaca ggtgactgcc ggcgtaagtc 1140
ggaggaaggc ggggatgacg tcaaatcatc atgcccctga tgtcctgggc tacacacgtg 1200
ctacaatggg cggtacaaag ggaggcgaag ccgcgaggcg gagcgaaacc caaaaagccg 1260
ctcgtagttc ggattgcagg ctgcaactcg cctgcatgaa gccggaattg ctagtaatcg 1320
cggatcagca tgccgcggtg aatacgttcc cgggccttgt acacaccgcc cgtcacacca 1380
```

```
cgagagtcgg caacacccga agtcggtgag gtaaccccga aaggggagcc agccgccgaa 1440
ggtggggtcg atgattgggg tgaagtcgta acaaggtagc cgtaccggaa ggtgc      1495
```